# EUROPEAN PATENT APPLICATION

(11) **EP 2 982 406 A1**
(43) Date of publication of application: **10.02.2016**
(21) Application number: 13881202.9
(22) Date of filing: 01.04.2013
(51) Int. Cl.: A61M 25/09

(54) **GUIDE WIRE**

(71) Applicant: Terumo Kabushiki Kaisha, Tokyo 151-0072 (JP)
(72) Inventor: TANO, Yutaka, Fujinomiya-shi Shizuoka 418-0015 (JP)
(74) Representative: Casalonga
(86) International application number: PCT/JP2013/059834
(87) International publication number: WO 2014/162393

(57) **Abstract**

Disclosed is a guide wire 1 including: a wire main body 11 that has flexibility; a distal end member 4 that is fixed to a distal end portion of the wire main body 11; and a tubular member 5 that is provided closer to a proximal end side than the distal end member 4, and on an outer circumference of the wire main body. The tubular member 5 is provided such that the longitudinal entirety of the tubular member 5 is not fixed to the wire main body 11. The tubular member 5 is provided such that a distal end portion of the tubular member 5 is not fixed to the distal end member 4. A proximal-end side thickness reduction portion 53 with a wall thickness decreasing toward the proximal end side is provided in a proximal end portion of the tubular member 5.

## Description

### Technical Field

The present invention relates to a guide wire.

### Background Art

A guide wire is used to guide a catheter which is used in the treatment of a portion on which it is difficult to perform surgery, in minimal invasive treatment for a human body, or in cardioangiographic examination, for example. For example, when percutaneous coronary intervention (PCI) is performed, a guide wire along with a balloon catheter under X-ray fluoroscopy is inserted into the vicinity of a stenosed site of a coronary artery, that is, a target portion, while a distal end of the guide wire protrudes further than a distal end of the balloon catheter, and a distal end portion of the balloon catheter is guided to the vicinity of a vascular stenosed site.

PTL 1 discloses a guide wire that is used in this type of treatment. This guide wire is configured to include a wire main body (core member) that has flexibility; a distal end member that is provided in a distal end portion of the wire main body; a coil (X-ray imaging metal coil) that is installed on a proximal end side of the distal end member so as to cover an outer circumference of the wire main body; and coating layers (coating member made of synthetic resin, and a hydrophilic lubricating layer) that cover the outermost surfaces of the wire main body and the coil. A distal end portion of the coil is fixed to the distal end member, and a proximal end portion of the coil is fixed to the wire main body.

When the guide wire disclosed in PTL 1 is operated as described above, phenomena to be described hereinbelow may occur depending on a state of the coronary artery, for example, the degree of curving.

For example, a portion (which includes the coil) of the guide wire may be caught by a vascular stenosed site that is formed on the wall of a blood vessel. Since the distal end portion of the coil is fixed to the distal end member, and the proximal end portion is fixed to the wire main body, even if the guide wire is rotated, torque is not sufficiently transmitted to a distal end portion of the guide wire, and operability of the guide wire considerably deteriorates, which is a problem.

### Citation List

### Patent Literature

[PTL 1] JP-A-2010-207251

### Summary of Invention

### Technical Problem

An object of the present invention is to provide a guide wire that can exhibit good operability even in a sharply curved blood vessel or in a vascular stenosed site.

### Solution to Problem

This object is achieved by (1) to (14) of the present invention.
(1) There is provided a guide wire including: a wire main body that has flexibility; a distal end member that is fixed to a distal end portion of the wire main body; and a tubular member that is provided closer to a proximal end side than the distal end member, and on an outer circumference of the wire main body, in which the tubular member is provided such that the longitudinal entirety of the tubular member is not fixed to the wire main body.
(2) In the guide wire according to (1), the tubular member is provided such that a distal end portion of the tubular member is not fixed to the distal end member.
(3) In the guide wire according to (1) or (2), a proximal-end side thickness reduction portion with a wall thickness decreasing toward the proximal end side is provided in a proximal end portion of the tubular member.
(4) In the guide wire according to (3), an outer-diameter increasing portion with an outer diameter increasing toward the proximal end side is provided in a middle of the wire main body in a longitudinal direction of the wire main body, and in a side view of the wire main body, at least a portion of the proximal-end side thickness reduction portion overlaps the outer-diameter increasing portion.
(5) In the guide wire according to any one of (1) to (4), a distal-end side thickness reduction portion with a wall thickness decreasing toward a distal end side is provided in the distal end portion of the tubular member.
(6) In the guide wire according to any one of (1) to (5), the tubular member is provided with a cut-away portion that decreases the rigidity of the tubular member.
(7) In the guide wire according to (6), the cut-away portion is a slit that is helically provided around a center axis of the tubular member.
(8) In the guide wire according to any one of (1) to (5), the tubular member is configured as a coil that is made by helically winding a wire rod.
(9) In the guide wire according to (8), the number of turns of the wire rod per unit length of the coil in at least one of a distal end portion and a proximal end portion of the coil is less than the number of turns of the wire rod per unit length in an intermediate portion between the distal end portion and the proximal end portion of the coil.
(10) In the guide wire according to any one of (1) to (9), the distal end member is formed in a cylindrical shape, and the inner diameter of the tubular member is smaller than the outer diameter of the distal end member.
(11) In the guide wire according to any one of (1) to (10), the longitudinal length of the tubular member is greater than that of the distal end member.
(12) In the guide wire according to any one of (1) to (11), a slide resistance reduction member is provided between the tubular member and the wire main body so as to reduce slide resistance between the tubular member and the wire main body.
(13) In the guide wire according to (12), the slide resistance reduction member is made of a hydrophilic material, fluorine-based resin, or silicon-based resin.
(14) In the guide wire according to any one of (1) to (13), the distal end member includes the coil that is made by helically winding the wire rod.

### Advantageous Effects of Invention

According to the present invention, it is possible to provide a guide wire that can exhibit good operability in a sharply curved blood vessel or in a vascular stenosed site.

In particular, in the present invention, a tubular member is provided such that the longitudinal entirety of the tubular member is not fixed to a wire main body, and the tubular member is provided such that a distal end portion of the tubular member is fixed or is not fixed to a distal end member. Accordingly, at least a proximal end portion of the tubular member can rotate relative to the wire main body.

### Brief Description of Drawings

[Fig. 1] Fig. 1 is a partial longitudinal sectional view (schematic side view) of a guide wire in a first embodiment of the present invention.
[Fig. 2] Fig. 2 is a longitudinal sectional view of a tubular member illustrated in Fig. 1.
[Fig. 3] Fig. 3 is a longitudinal sectional view of a tubular member of the guide wire in a second embodiment of the present invention.
[Fig. 4] Fig. 4 shows longitudinal sectional views of a tubular member of the guide wire in a third embodiment of the present invention, Fig. 4 (a) is a view illustrating a state in which the tubular member is caught by a vascular stenosed site, Fig. 4 (b) is a view illustrating a state in which a wire main body is rotated in the direction of arrow A in Fig. 4(a), and Fig. 4(c) is a view illustrating a state in which the wire main body is rotated in the direction of arrow B in Fig. 4(a) .
[Fig. 5] Fig. 5 is a longitudinal sectional view of a tubular member of the guide wire in a fourth embodiment of the present invention.

### Description of Embodiments

Hereinafter, preferred embodiments of a guide wire of the present invention will be described in detail with reference to the accompanying drawings.

### <First Embodiment>

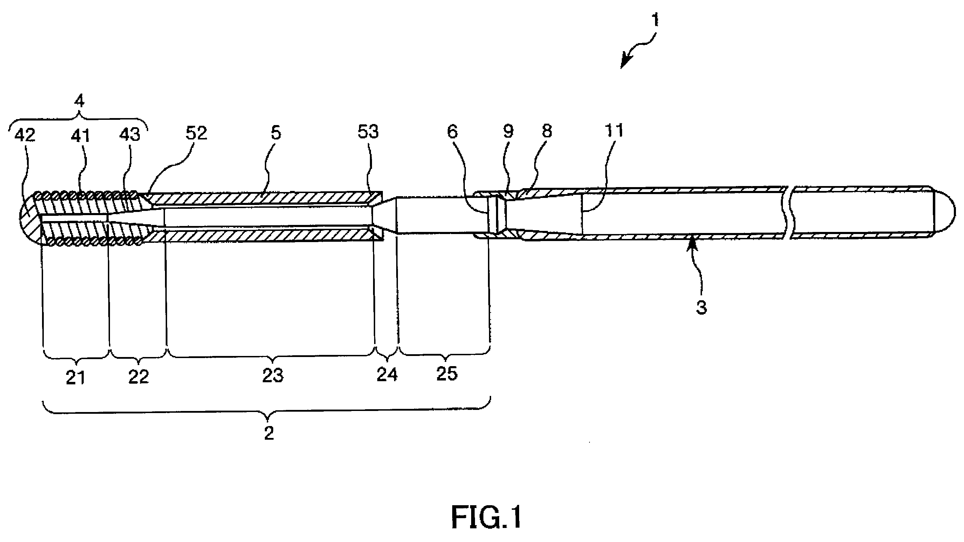

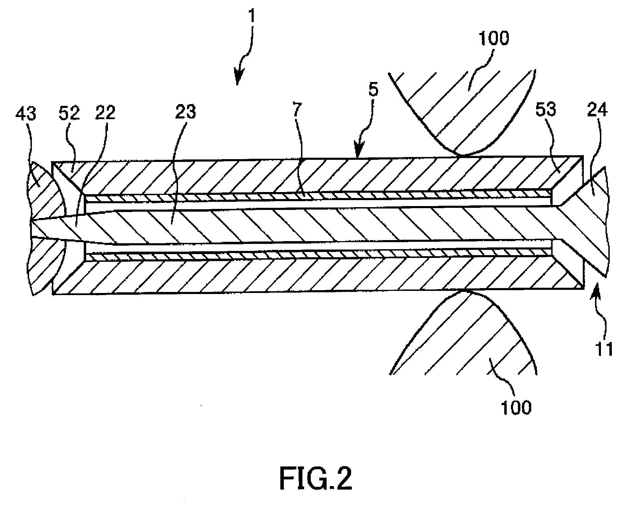

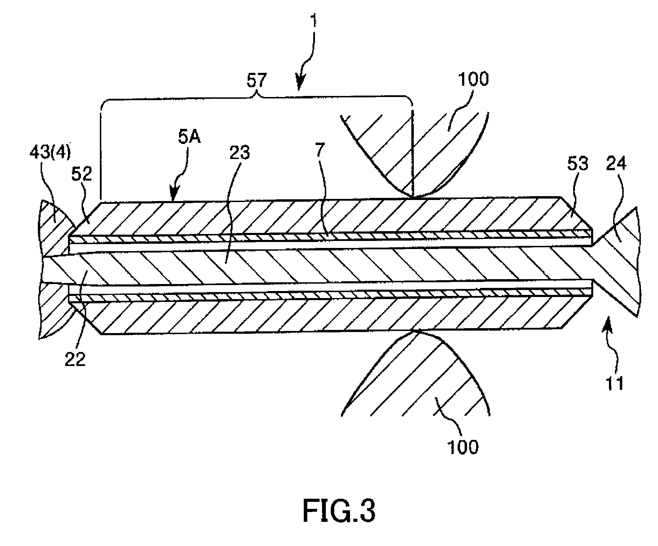

Fig. 1 is a partial longitudinal sectional view (schematic side view) of a guide wire in a first embodiment of the present invention, and Fig. 2 is a longitudinal sectional view of a tubular member illustrated in Fig. 1. Hereinafter, for illustrative purposes, in Figs. 1 and 2, the right side and the left side in a longitudinal direction are respectively referred to as a "proximal end" and a "distal end", and an upper side and a lower side are respectively referred to as the "top" and the "bottom". In Figs. 1 and 2, for ease of understanding, the guide wire is schematically illustrated in a state where the longitudinal length of the guide wire is decreased, and the radial length (thickness) of the guide wire is increased, and the ratio of the longitudinal length to the radial length is different from an actual ratio (the same also applies to Fig. 3 and the subsequent drawings). The guide wire of the present invention exhibits good operability in a vascular stenosed site in which the distance between the walls of a blood vessel is reduced, a sharply curved blood vessel, and the like, and hereinafter, a case, in which the guide wire of the present invention is positioned in a vascular stenosed site, will be representatively described.

A guide wire 1 illustrated in Fig. 1 is a guide wire for a catheter (or an endoscope) which is used while being inserted into the lumen of a catheter, and includes a flexible wire main body 11 in which a second wire 3 disposed on a proximal end side of a first wire 2 is joined (connected) to the first wire 2 that is disposed on a distal end side; a distal end member 4 that has a coil 41 which is fixed to a distal end portion of the wire main body 11 using fixing members 42 and 43; and a tubular member 5 that is provided on a proximal end side of the coil 41 and on an outer circumference of the wire main body 11. The entire length of the guide wire 1 is not limited to a specific dimension, and the guide wire 1 preferably has a total length of approximately 200 mm to approximately 5000 mm. The outer diameter of the guide wire 1 is not limited to a specific dimension, and typically, the guide wire 1 has an outer diameter of approximately 0.2 mm to approximately 1.2 mm.

The first wire 2 is made of a wire rod (core member) with flexibility or elasticity. The length of the first wire 2 is not limited to a specific dimension, and the first wire 2 preferably has a length of approximately 20 mm to approximately 1000 mm.

In the embodiment, the first wire 2 has a portion (constant outer-diameter portion) that has a constant outer diameter, and a tapered portion (gradual outer-diameter reduction portion), the outer diameter of which gradually decreases toward a distal end thereof. In an illustrated configuration, the first wire 2 has a constant outer-diameter portion 25; a tapered portion (outer-diameter increasing portion) 24; a constant outer-diameter portion 23 with an outer diameter which is smaller than that of the constant outer-diameter portion 25; a tapered portion (main body-side tapered portion) 22; and a foremost end portion 21, all of which are sequentially disposed from a proximal end side to the distal end side.

Since the guide wire 1 has the tapered portions 22 and 24, it is possible to gradually decrease the rigidity (flexural rigidity and torsional rigidity) of the first wire 2 toward the distal end. As a result, a distal end portion of the guide wire 1 can have good flexibility, the guide wire 1 can have an improved ability of being able to follow a body lumen (body cavity) such as a blood vessel or the like, and improved safety, and can be prevented from being bent, for example.

The taper angle (reduction rate in the outer diameter) of each of the tapered portions 22 and 24 may be constant or changed along a longitudinal direction (hereinafter, which is simply referred to as a "longitudinal direction") of the wire main body 11. For example, regions formed with a relatively large taper angle (reduction rate in the outer diameter) and regions formed with a relatively small taper angle may be alternately repeated multiple times.

For example, the foremost end portion 21 can be configured as a constant outer-diameter portion that has an outer diameter which is smaller than that of the constant outer-diameter portion 23.

For example, the foremost end portion 21 is formed in the shape of a flat plate (ribbon shape), and it is possible to use the foremost end portion 21 in a state where the shape of the foremost end portion 21 is changed (re-shaped or shaped) to a desired shape. Typically, a doctor uses the guide wire in a state where the distal end portion of the guide wire is bent in a predetermined desired shape so that a distal end portion of a guiding catheter or the like can be adapted to the shape of a blood vessel, or can be smoothly guided to a blood vessel branch. As such, the bending of the distal end portion of the guide wire in a desired shape is referred to as the term "re-shaping". Since the foremost end portion 21 is provided, it is possible to easily and reliably re-shape the guide wire 1, and to considerably improve operability when the guide wire 1 is inserted into a living body.

The length of the foremost end portion 21 is not limited to a specific dimension, and the foremost end portion preferably has a length of approximately 5 mm to approximately 200 mm, and more preferably has a length of approximately 10 mm to approximately 150 mm. In particular, in a case where the foremost end portion 21 is re-shaped and is then used, when the length of the foremost end portion 21 is excessively long, operability of the guide wire 1 deteriorates due to the material of the foremost end portion 21, and when the length of the foremost end portion 21 is excessively short, the distal end portion of the guide wire 1 cannot be formed in a desired shape, which is a problem.

The material (wire) of the first wire 2 is not limited to a specific material, and various metal materials such as an Ni-Ti alloy, stainless steel or the like can be used as the material of the first wire 2, and preferably, alloys with pseudoelasticity (including a superelastic alloy) are used. A superelastic alloy is more preferably used. Since the first wire 2 is made of the superelastic alloy which is relatively flexible, has resilience, and is unlikely to have a tendency to bend, the distal end portion of the guide wire 1 can have sufficient flexibility, and resilience against bending. Therefore, it is possible to obtain an improved ability of being able to follow complex curved and bent blood vessels, and to obtain better operability. Since the first wire 2 is unlikely to have a tendency to bend due to the resilience of the first wire 2, even if the first wire 2 repeatedly undergoes curving and bending deformation, the tendency to bend of the first wire 2 of the guide wire 1 during use can be prevented from causing deterioration in the operability.

The pseudoelastic alloys include pseudoelastic alloys with any tensile stress-strain curves, pseudoelastic alloys in which the transformation point of As, Af, Ms, Mf, or the like can be or cannot be measured in a distinguishing manner, and pseudoelastic alloys which are considerably deformed (distorted) due to stress, and substantially return to their original shape when the stress is removed.

With regard to the compositions of the superelastic alloys exemplified herein, preferably, a Ni-Ti alloy such as a Ni-Ti alloy containing 49% to 52% of Ni atoms or the like, a Cu-Zn alloy containing 38.5% to 41.5% by weight of Zn, a Cu-Zn-X alloy containing 1% to 10% by weight of X (X is at least one type of Be, Si, Sn, Al, and Ga), a Ni-Al alloy containing 36% to 38% of Al atoms and the like are used. In particular, the Ni-Ti alloy among these alloys is preferably used. The superelastic alloys represented by the Ni-Ti alloy have good adhesion to a resin coating layer when the guide wire 1 is used while being coated with the resin coating layer.

A distal end of the second wire 3 is joined (connected) to a proximal end of the first wire 2 (proximal end of the constant outer-diameter portion 25). The second wire 3 is made of a wire rod (core member) with flexibility or elasticity. The length of the second wire 3 is not limited to a specific dimension, and the second wire 3 preferably has a length of approximately 20 mm to approximately 4800 mm, and more preferably has a length of approximately 1400 mm to approximately 3000 mm.

The mean outer diameter of the second wire 3 is greater than that of the first wire 2. Accordingly, since the guide wire 1 is configured such that the first wire 2 on the distal end side of the guide wire 1 has good flexibility, and the second wire 3 on the proximal end side thereof has high rigidity, the flexibility of the distal end portion can be compatible with good operability (pushing performance, torque transmission performance, and the like).

A method of joining the first wire 2 to the second wire 3 is not limited to a specific method, and it is possible to use, for example, various methods such as welding, soldering or the like. The first wire 2 is preferably joined to the second wire 3 using welding.

The welding method is not limited to a specific method, and friction pressure welding, spot welding using a laser beam, butt resistance welding such as upset welding or the like, or the like can be used. In particular, the butt resistance welding is preferably used because it is possible to relatively simply obtain high joining strength.

The second wire 3 is made of a material that is different from that of the first wire 2, and particularly, the second wire 3 is preferably made of a material, the elastic modulus (Young's modulus (modulus of longitudinal elasticity), the modulus of rigidity (modulus of transverse elasticity), or the bulk modulus) of which is higher than that of the first wire 2. Accordingly, the second wire 3 can have appropriate rigidity (flexural rigidity and torsional rigidity), and the guide wire 1 is rigid, and as a result, it is possible to obtain improved pushing performance and torque transmission performance, and to obtain better ease of insertion.

The material (wire) of the second wire 3 is not limited to a specific material insofar as the material of the second wire 3 is different from that of the first wire 2, and it is possible to use various metal materials such as stainless steel (all types of SUS such as SUS304, SUS303, SUS316, SUS316L, SUS316J1, SUS316J1L, SUS405, SUS430, SUS434, SUS444, SUS429, SUS430F, SUS302, or the like), piano wire, cobalt alloys, alloys with pseudoelasticity, and the like. Stainless steel or a cobalt alloy is preferably used as the material of the second wire 3, and stainless steel is more preferably used. Since the second wire 3 is made of stainless steel or a cobalt alloy, the guide wire 1 can have good pushing performance and torque transmission performance.

In the embodiment, the wire main body 11 is configured such that the first wire 2 is joined to the second wire 3; however, the present invention is not limited to that configuration in the embodiment, and for example, the wire main body 11 may be configured as a single continuous wire rod.

As illustrated in Fig. 1, the coil 41, the outer diameter and the inner diameter of which are constant, is installed on an outer circumference of the distal end portion of the wire main body 11, that is, on the outer circumferences of the foremost end portion 21 and the tapered portion 22 of the first wire 2. The coil 41 is a member that is made by helically winding a wire rod 40, and is installed in such a way as to cover the distal end portion of the wire main body 11, that is, the foremost end portion 21 and a portion of the tapered portion 22 excluding a proximal end portion of the tapered portion 22 in the first wire 2. The first wire 2 is inserted into substantially the inner center portion of the coil 41 while not being in contact with the coil 41.

The coil 41 preferably has a length (the longitudinal length of the wire main body 11) of 5 mm to 500 mm, and more preferably has a length of 10 mm to 300 mm. The coil 41 preferably has an inner diameter of 0.1 mm to 0.95 mm, and more preferably has an inner diameter of 0.2 mm to 0.7 mm.

The coil 41 is preferably made of a metal material. Stainless steel, superelastic alloys, cobalt alloys, noble metals such as gold, platinum, tungsten or the like, and alloys (for example, a platinum-iridium alloy) containing these noble metals, or the like can be used as the metal material of the coil 41. In particular, when the coil 41 is made of a radiopaque material such as noble metals, the guide wire 1 can be X-ray imaged, and it is possible to insert the guide wire 1 into the living body while confirming the position of the distal end portion under X-ray fluoroscopy, which is preferable. A distal end side and a proximal end side of the coil 41 may be made of different materials. For example, the distal end side of the coil may be made of a radiopaque material, and the proximal end side of the coil may be made of a material (stainless steel or the like) through which X rays are transmitted relatively well.

A distal end portion and a proximal end portion of the coil 41 are fixed to the first wire 2 using the fixing members 42 and 43, respectively. Solder (brazing material) is used as the fixing members 42 and 43. The fixing members 42 and 43 are not limited to solder, and may be adhesives. A method of fixing the coil 41 to the first wire 2 is not limited to the use of these fixing members, and may be welding. A distal end surface of the fixing member 42 is preferably rounded so as to prevent an inner wall of a body cavity such as a blood vessel or the like from being damaged.

In the embodiment, since the coil 41 is installed in this way, the distal end portion of the guide wire 1 can have appropriate flexibility, and since the first wire 2 is covered with the coil 41, and has a small contact area, it is possible to reduce the slide resistance of the first wire 2, and the operability of the guide wire 1 is further improved.

The tubular member 5, which is formed as a tube, is provided on the proximal end side of the coil 41 and on the outer circumference of the wire main body 11. The tubular member 5 has a distal-end side thickness reduction portion 52 that is provided in a distal end portion of the tubular member 5, and a proximal-end side thickness reduction portion 53 that is provided on a proximal end portion thereof.

The tubular member 5 has a circular cross section, and is non-fixedly inserted onto the wire main body 11 in such a way as to cover a proximal end portion of the tapered portion 22, the constant outer-diameter portion 23, and a distal end portion of the tapered portion 24. The tubular member 5 is provided in such a way that the longitudinal entirety of the tubular member 5 is not fixed to the wire main body 11, and is not fixed to the distal end member 4 (the coil 41 and the fixing member 43). Accordingly, the longitudinal entirety of the tubular member 5 can rotate independently of the wire main body 11 and the coil 41. As a result, even if the tubular member 5 is caught by a vascular stenosed site 100 that is formed on the wall of a blood vessel, when a rotating force is applied to a proximal end portion of the wire main body 11, the rotating force can be sufficiently transmitted to the distal end portion of the wire main body 11, and thus the wire main body 11 can rotate relative to the fixed tubular member 5. Accordingly, the guide wire 1 can exhibit good operability in a sharply curved blood vessel or the vascular stenosed site 100.

The distal-end side thickness reduction portion 52 is a portion in which the wall thickness of the tubular member 5 decreases toward the distal end side. That is, the distal-end side thickness reduction portion 52 is a portion in which the outer diameter of the tubular member 5 is constant, and the inner diameter of the tubular member 5 increases toward the distal end side. Since the distal-end side thickness reduction portion 52 is provided, it is possible to decrease flexural rigidity of the distal end portion of the tubular member 5. Accordingly, flexibility of the distal-end side thickness reduction portion 52 is improved. Even if the wire main body 11 is sharply curved, the distal end portion of the tubular member 5 can be deformed to reliably follow the wire main body 11. As a result, the deformation of the wire main body 11 cannot be prevented.

In a side view of the wire main body 11, the distal-end side thickness reduction portion 52 overlaps a proximal end portion of the distal end member 4 (a part of the fixing member 43 and a part of the coil 41). That is, the distal-end side thickness reduction portion 52 is provided so as to cover a rounded outer circumferential surface of the fixing member 43. Accordingly, it is possible to reduce the space between the distal end member 4 and the tubular member 5. As a result, a step at the boundary between the distal end member 4 and the tubular member 5 is reduced, and the guide wire 1 can be prevented from being caught by the wall of a blood vessel, for example.

The proximal-end side thickness reduction portion 53 is a portion in which the wall thickness of the tubular member 5 decreases toward the proximal end side. That is, the proximal-end side thickness reduction portion 53 is a portion in which the outer diameter of the tubular member 5 is constant, and the inner diameter of the tubular member 5 increases toward the proximal end side. Since the proximal-end side thickness reduction portion 53 is provided, it is possible to decrease flexural rigidity of the proximal end portion of the tubular member 5. Accordingly, flexibility of the proximal-end side thickness reduction portion 53 is improved. Even if the wire main body 11 is sharply curved, the proximal end portion of the tubular member 5 can be deformed to reliably follow the wire main body 11. As a result, the deformation of the wire main body 11 can be prevented.

In a side view of the wire main body 11, the proximal-end side thickness reduction portion 53 overlaps the tapered portion 24 of the wire main body 11. As described above, since the proximal-end side thickness reduction portion 53 has high flexibility, the tapered portion 24 can provide flexibility to the wire main body 11. The proximal-end side thickness reduction portion 53 is provided so as to cover the outer surface of the tapered portion 24. Accordingly, it is possible to reduce the space between the proximal end portion of the tubular member 5 and the tapered portion 24 of the wire main body 11. As a result, a step at the boundary between the proximal-end side thickness reduction portion 53 and the tapered portion 24 is reduced, and the guide wire 1 can be prevented from being caught by the wall of a blood vessel, for example.

The tubular member 5 preferably has an outer diameter of 0.2 mm to 1.0 mm, and more preferably has an outer diameter of 0.36 mm to 0.89 mm. The tubular member 5 preferably has an inner diameter of 0.1 mm to 0.95 mm, and more preferably has an inner diameter of 0.2 mm to 0.7 mm. Accordingly, the inner diameter of the tubular member 5 is much smaller than the outer diameter of the coil 41, and thus the distal end portion of the tubular member 5 is restricted from moving further toward the distal end side than the coil 41. Accordingly, the tubular member 5 can be prevented from moving upward relative to the distal end member 4.

In addition, the inner diameter of the tubular member 5 is less than the maximum value of the outer diameter of the tapered portion 24 of the wire main body 11. Accordingly, the tapered portion 24 restricts the proximal end portion of the tubular member 5 from moving further toward the proximal end side than the tapered portion 24. As a result, the tubular member 5 can be prevented from moving upward relative to the tapered portion 24 and the constant outer-diameter portion 25.

Since the tubular member 5 is restricted from moving toward the distal end side and the proximal end side, even if the guide wire 1 passes through a relatively narrow site such as the vascular stenosed site 100, the tubular member 5 can remain between the fixing member 43 and the tapered portion 24.

The length of the tubular member 5 is preferably greater than that of the coil 41. Specifically, the tubular member 5 preferably has a length of 10 mm to 500 mm, and more preferably has a length of 50 mm to 300 mm. The length of the tubular member 5 is preferably 0.3% to 30% of the length of the wire main body 11, and is more preferably 1.5% to 15% of the length of the wire main body 11. Accordingly, in the guide wire 1, the tubular member 5 can receive the vascular stenosed site 100 as much as possible. When the guide wire 1 is used, the tubular member 5 is brought into a state of being inserted into a body lumen such as a blood vessel or the like.

A slide resistance reduction member is provided between the tubular member 5 and the wire main body 11 (the constant outer-diameter portion 23), and reduces slide resistance between the tubular member 5 and the wire main body 11. In the embodiment, the inner circumferential surface of the tubular member 5 is coated with a hydrophilic lubricating layer 7 that is made of a hydrophilic material (refer to Fig. 2). Accordingly, lubricating performance is obtained due to wetness of the hydrophilic material, the friction (slide resistance) between the tubular member 5 and the wire main body 11 is reduced, and sliding performance is improved.

The following materials are exemplified as the hydrophilic materials (the material of the hydrophilic lubricating layer 7): a cellulosic polymer, a polyethylene oxide polymer; a maleic anhydride polymer (for example, maleic anhydride copolymer such as methyl vinyl ether-maleic anhydride copolymer); an acrylamide polymer (for example, polyacrylamide, and polyglycidyl methacrylate-dimethyl acrylamide (PGMA-DMAA) block copolymer) ; water-soluble nylon; polyvinyl alcohol; polyvinylpyrrolidone; and the like.

In the embodiment, the hydrophilic lubricating layer 7 is provided on the inner circumferential surface of the tubular member 5; however, the hydrophilic lubricating layer 7 may be provided on the entire outer surface of the wire main body 11. The hydrophilic lubricating layer 7 may be provided on the outer surface of the tubular member 5. Accordingly, it is possible to reduce friction resistance (slide resistance) between the tubular member 5 and the inner wall of the catheter that is used along with the guide wire 1. As a result, the sliding performance of the guide wire 1 is improved, and the operability of the guide wire 1 in the catheter is further improved.

In the aforementioned example, the slide resistance reduction member is made of the hydrophilic material; however, the present invention is not limited to the use of the hydrophilic material, and the slide resistance reduction member may be made of fluorine-based resin, silicon-based resin, or the like. The following materials are exemplified as the fluorine-based resin: polytetrafluoroethylene (PTFE); ethylene tetrafluoroethylene copolymer (ETFE); tetrafluoroethylene-perfluoroalkyl vinyl ether copolymer (PFA) ; and the like. Examples of the silicon-based resin are: silicone resin and the like. In addition, a composite material, which is a combination of these materials, may be used.

The guide wire 1 has resin coating layers 8 and 9 which entirely or partially cover the outer circumferential surface (outer surface) of the wire main body 11. In an illustrated configuration, the resin coating layer 9 is provided on an outer circumference of a joined portion 6 of the wire main body 11, and the resin coating layer 8 is provided on an outer circumferential portion that is positioned closer to the proximal end side than the joined portion 6.

The resin coating layers 8 and 9 can be formed for various purposes, and for example, due to the formation of the resin coating layers 8 and 9, the friction (slide resistance) of the guide wire 1 is reduced, and sliding performance is improved, and thus the operability of the guide wire 1 is improved.

In order to reduce the friction (slide resistance) of the guide wire 1, the resin coating layers 8 and 9 are preferably made of friction reduction materials which will be described hereinbelow. Accordingly, friction resistance (slide resistance) between the guide wire 1 and the inner wall of the catheter in use therewith is reduced, and thus the sliding performance of the guide wire 1 is improved, and the operability of the guide wire 1 in the catheter is further improved. Since the slide resistance of the guide wire 1 is reduced, it is possible to more reliably prevent the occurrence of a kink (bending) or twist in the guide wire 1, and particularly, the occurrence of a kink or twist in the vicinity of a joined portion (joined surface) 6 between the first wire 2 and the second wire 3 when the guide wire 1 moves and/or rotates in the catheter.

The following materials are exemplified as the material of the resin coating layers 8 and 9: polyolefin such as polyethylene, polypropylene or the like; polyvinyl chloride; polyester (PET, PBT, or the like); polyamide; polyimide; polyurethane; polystyrene; polycarbonate; silicone resin; fluorine-based resin (PTFE, ETFE, or the like) ; and a composite material which is a combination of these materials.

In particular, when fluorine-based resin (or a composite material containing fluorine-based resin) among these materials is used, it is possible to effectively reduce the friction resistance (slide resistance) between the guide wire 1 and the inner wall of the catheter, and thus it is possible to improve sliding performance, and the operability of the guide wire 1 in the catheter is further improved. Accordingly, it is possible to more reliably prevent the occurrence of a kink (bending) or twist in the guide wire 1, and particularly, the occurrence of a kink or twist in the vicinity of joined portions when the guide wire 1 moves and/or rotates in the catheter.

When fluorine-based resin (or a composite material containing fluorine-based resin) is used, the wire main body 11 can be coated with the resin material using baking, spraying, or the like in a state where the resin material is heated. Accordingly, adhesion between the wire main body 11 and the resin coating layers 8 and 9 is considerably good.

In a case where the resin coating layers 8 and 9 are made of silicone resin (or a composite material containing silicone resin), even if the silicone resin is not heated when the resin coating layers 8 and 9 are formed (the wire main body 11 is coated with the resin coating layers 8 and 9), it is possible to form the resin coating layer 8 that reliably and strongly adheres to the wire main body 11. That is, in a case where the resin coating layers 8 and 9 are made of silicone resin (or a composite material containing silicone resin), since a reaction curable resin material or the like can be used, the resin coating layers 8 and 9 can be formed at room temperature. Since the resin coating layers 8 and 9 are formed at room temperature, it is possible to simply perform coating, and to operate the guide wire in a state where sufficient joining strength of the joined portion 6 is maintained.

The material of the resin coating layer 8 may be the same as or different from that of the resin coating layer 9.

The thickness of each of the resin coating layers 8 and 9 is not limited to a specific dimension, and is appropriately determined while the purpose and the method of formation of the resin coating layer 8, the material of the resin coating layer 8, and the like are taken into consideration, and typically, the resin coating layer 8 preferably has a thickness (mean thickness) of approximately 1 µm to approximately 100 µm, and more preferably has a thickness of approximately 1 µm to approximately 30 µm. When the thickness of each of the resin coating layers 8 and 9 is excessively small, the purpose of formation of each of the resin coating layers 8 and 9 may not be sufficiently shown, and separation of the resin coating layers 8 and 9 may occur, which are problems. When the thickness of each of the resin coating layers 8 and 9 is excessively large, physical characteristics of the wire main body 11 may be affected, and separation of the resin coating layer 8 may occur, which are problems.

Each of the resin coating layers 8 and 9 may be a single layer, or may be a laminated body with two or more layers.

In the present invention, treatment (surface roughening, chemical treatment, heat treatment, or the like) can be applied to the outer circumferential surface (surface) of the wire main body 11 so as to improve adhesion of the resin coating layers 8 and 9, or an intermediate layer can be provided on the outer circumferential surface of the wire main body 11 so as to improve adhesion of the resin coating layers 8 and 9.

### <Second Embodiment>

Fig. 3 is a longitudinal sectional view of a tubular member of the guide wire in a second embodiment of the present invention.

Hereinafter, the guide wire of the second embodiment of the present invention will be described with reference to this drawing. The description will be centered around the points of difference between the first embodiment and the second embodiment, and the same items will not be described. For illustrative purposes, in Fig. 3, the right side and the left side are respectively referred to as a "proximal end" and a "distal end", and an upper side and a lower side are respectively referred to as the "top" and the "bottom".

This embodiment is the same as the first embodiment except that the shape of the tubular member is different from that in the first embodiment.

As illustrated in Fig. 3, a tubular member 5A in the embodiment has the distal-end side thickness reduction portion 52 and the proximal-end side thickness reduction portion 53.

The distal-end side thickness reduction portion 52 is a portion in which the inner diameter of the tubular member 5A is constant, and the outer diameter of the tubular member 5A gradually decreases toward the proximal end side. The distal-end side thickness reduction portion 52 and the tapered portion 22 are collected and fixed using the fixing member 43. Accordingly, a distal end portion of the tubular member 5A is fixed to the distal end portion 4. As a result, the tubular member 5A can remain between the fixing member 43 and the tapered portion 24.

The proximal-end side thickness reduction portion 53 is a portion in which the inner diameter of the tubular member 5A is constant, and the outer diameter of the tubular member 5A gradually decreases toward the proximal end side.

A proximal end portion of the tubular member 5A is provided while not being fixed to the wire main body 11. Accordingly, the proximal end portion of the tubular member 5A can rotate relative to the wire main body 11. As a result, even if the tubular member 5A is caught by the vascular stenosed site 100 that is formed on a blood vessel, when the proximal end portion of the guide wire 1 is gripped and rotated, a rotating force is transmitted to the tubular member 5A via the fixing member 43. Accordingly, a portion 57 is twisted, and thus the rotating force is transmitted to the distal end of the guide wire 1.

In the embodiment, the movement of the tubular member 5A is more reliably restricted, and the guide wire 1 can show good operability even in the vascular stenosed site 100.

### <Third Embodiment>

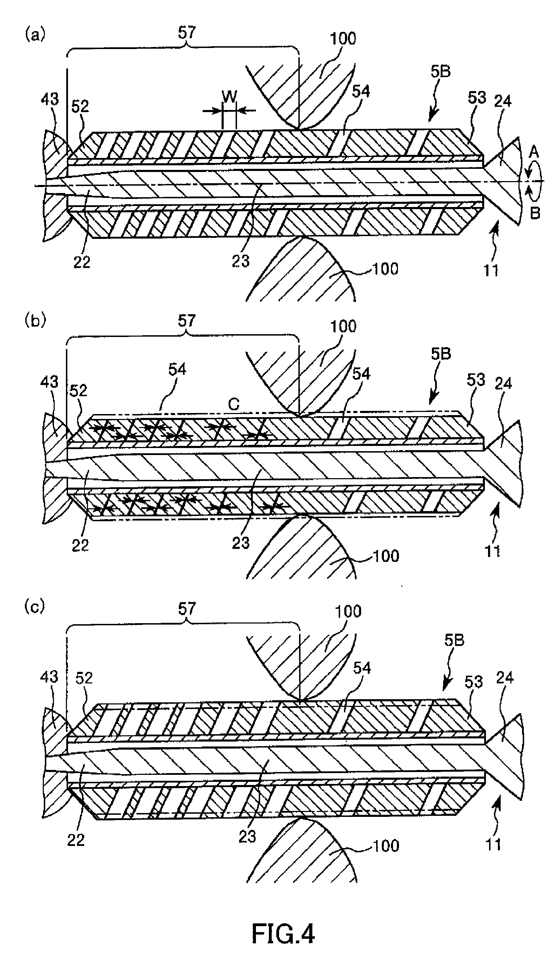

Fig. 4 shows longitudinal sectional views of a tubular member of the guide wire in a third embodiment of the present invention.

Hereinafter, the guide wire of the third embodiment of the present invention will be described with reference to this drawing. The description will be centered around the points of difference between the first embodiment and the third embodiment, and the same items will not be described. For illustrative purposes, in Fig. 4, the right side and the left side are respectively referred to as a "proximal end" and a "distal end", and an upper side and a lower side are respectively referred to as the "top" and the "bottom". This embodiment is the same as the second embodiment except that the shape of the tubular member is different from that in the

### second embodiment.

As illustrated in Fig. 4, a tubular member 5B is provided with a single continuous slit 54 that is a cut-away portion to reduce the rigidity of the tubular member 5B.

The slit 54 is helically provided around the center axis of the tubular member 5B. The slit 54 passes through the tubular member 5B from the outer circumferential surface of the tubular member 5B to the inner circumferential surface thereof, and a width W of the slit 54 is constant over the entire length of the slit 54. The width W of the slit 54 is preferably 0.001 mm to 1.0 mm, and is more preferably 0.002 mm to 0.5 mm. In the embodiment, the width W of the slit is constant over the entire length of the slit; however, the width W of the slit may be changed over the entire length of the slit.

As illustrated in Fig. 4 (a), during the use of the guide wire 1, the tubular member 5B may be brought into a state in which the tubular member 5B is caught and is fixed by the vascular stenosed site 100 on a blood vessel. In this state, when the proximal end portion of the guide wire 1 is gripped and rotated in the direction of arrow A in Fig. 4 (a), a rotating force is transmitted to the tubular member 5B via the fixing member 43. Accordingly, the portion 57 is twisted, and thus the outer diameter of the portion 57 decreases. In the portion 57, the gap between the adjacent slits 54 is reduced in directions of arrows illustrated in Fig. 4(b), and the width of the slit 54 is further reduced than the width (alternate one long and two short dashes line in Fig. 4(b)) of the slit 54 in Fig. 4(a).

In contrast, when the guide wire 1 is rotated in the direction of arrow B in Fig. 4(c) from a state illustrated in Fig. 4 (a), reversely to the former case, the width of the slit 54 is further increased than the width (alternate one long and two short dashes line in Fig. 4 (c)) of the slit 54 in Fig. 4 (a).

The slits 54 are densely formed toward the distal end side. Accordingly, a distal end portion of the tubular member 5B has flexural rigidity and torsional rigidity that are lower than those of a proximal end portion of the tubular member 5B. As a result, the distal end portion of the tubular member 5B has good flexibility, is more easily twisted, and can be easily rotated.

In the embodiment, since the rigidity of the tubular member 5B is reduced, the operability of the guide wire 1 is further improved. It is possible to obtain desired characteristics by changing an interval at which the slits 54 are formed as necessary. For example, it is possible to obtain the same effects as those in the first embodiment by densely forming the slits 54 in the distal end portion and the proximal end portion of the tubular member 5B, and non-densely forming the slits 54 in an intermediate portion between the distal end portion and the proximal end portion.

### <Fourth Embodiment>

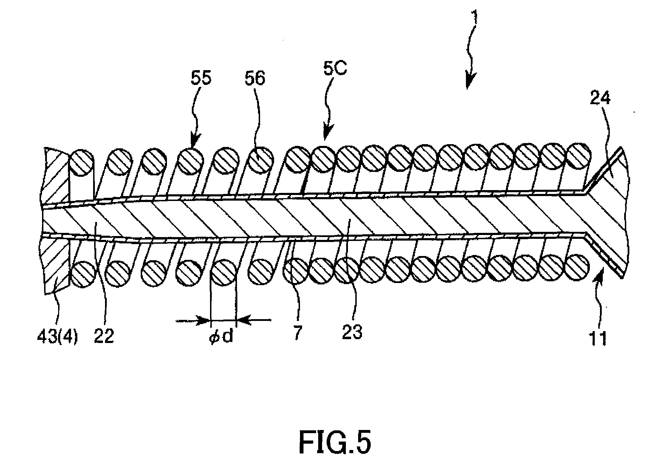

Fig. 5 is a longitudinal sectional view of a tubular member of the guide wire in a fourth embodiment of the present invention.

Hereinafter, the guide wire of the fourth embodiment of the present invention will be described with reference to this drawing. The description will be centered around the points of difference between the first embodiment and the fourth embodiment, and the same items will not be described. For illustrative purposes, in Fig. 5, the right side and the left side are respectively referred to as a "proximal end" and a "distal end", and an upper side and a lower side are respectively referred to as the "top" and the "bottom".

This embodiment is the same as the third embodiment except that the shape of the tubular member is different from that in the third embodiment.

A tubular member 5C of the embodiment is configured as a coil with a constant outer diameter and a constant inner diameter. A coil 55 is formed by helically winding a wire rod 56 with a circular cross section. The number of turns of the wire rod 56 per unit length (the longitudinal length of the wire main body 11) in a distal end portion of the coil 55 is less than the number of turns of the wire rod 56 per unit length in a proximal end portion and an intermediate portion of the coil 55. Accordingly, it is possible to reduce the rigidity of the distal end portion of the coil 55.

Similar to the distal end portion of the coil 55, the number of turns of the wire rod 56 per unit length in the proximal end portion of the coil 55 is less than the number of turns of the wire rod 56 per unit length in the intermediate portion of the coil 55. Accordingly, it is possible to reduce the rigidity of the proximal end portion of the coil 55.

A diameter φd of the wire rod 56 is preferably 0.01 mm to 0.2 mm, and is more preferably 0.04 mm to 0.1 mm. Accordingly, in the coil 55, the flexibility can be compatible with the rigidity.

It is possible to provide a distal-end side thickness reduction portion of the coil 55 by reducing the wire diameter (wall thickness of the tubular member) of the wire rod 56 of the coil 55 toward the distal end portion so as to increase flexibility of the end portion. In this case, it is possible to provide the distal-end side thickness reduction portion by reducing the outer diameter of the coil 55 toward the distal end side while keeping the inner diameter of the coil 55 constant, or by increasing the inner diameter of the coil 55 toward the distal end side while keeping the outer diameter of the coil 55 constant.

Similarly, it is possible to provide a proximal-end side thickness reduction portion of the coil 55 by reducing the wire diameter of the wire rod 56 of the coil 55 toward the proximal end portion. In this case, it is possible to provide the proximal-end side thickness reduction portion by reducing the outer diameter of the coil 55 toward the proximal end side while keeping the inner diameter of the coil 55 constant, or by increasing the inner diameter of the coil 55 toward the proximal end side while keeping the outer diameter of the coil 55 constant. In a side view of the wire main body 11, the proximal-end side thickness reduction portion overlaps the tapered portion 24 of the wire main body 11.

The material of the coil 55 may be the same as that of the coil 41 of the distal end member 4. The material of the coil 41 may be the same as or different from that of the coil 55.

In the embodiment, the hydrophilic lubricating layer 7 is provided on the outer circumferential surface of the wire main body 11. Specifically, the hydrophilic lubricating layer 7 is provided on the outer circumferential surfaces of the tapered portion 22, the constant outer-diameter portion 23, and the tapered portion 24.

In the tubular member 5 that is configured as the coil 55, it is possible to obtain the same effects as those in the third embodiment.

The guide wire of the present invention has been described based on the illustrated embodiments; however, the present invention is not limited to those in the embodiments, and configurational elements can be replaced with arbitrary elements having the same functions. Other arbitrary configurational elements may be added to the present invention.

In the embodiments, a stopper or the like for restricting the movement of the tubular member in the longitudinal direction may be separately provided.

The cut-away portion in the third embodiment is configured as a slit; however, the present invention is not limited to that configuration, and the cut-away portion may be, for example, a plurality of through holes which are formed in the wall of the tubular member; a groove that opens toward an outer circumferential side or an inner circumferential side; or the like.

In the third embodiment, a single continuous slit is provided; however, the present invention is not limited to that configuration, and a plurality of slits may be formed.

In the fourth embodiment, the wire rod of the coil has a circular cross section; however, the present invention is not limited to that shape, and the wire rod of the coil may have, for example, a semicircular cross section, a flat cross section, or the like.

### Industrial Applicability

A guide wire of the present invention has a wire main body that has flexibility; a distal end member that is fixed to a distal end portion of the wire main body; and a tubular member that is provided closer to a proximal end side than the distal end member, and on an outer circumference of the wire main body. The tubular member is provided such that the longitudinal entirety of the tubular member is not fixed to the wire main body, and the tubular member is provided such that a distal end portion of the tubular member is fixed or is not fixed to the distal end member. Accordingly, at least a proximal end portion of the tubular member is configured to be able to rotate relative to the wire main body.

According to the present invention, it is possible to provide the guide wire that exhibits good operability even in a sharply curved blood vessel, a vascular stenosed site, or the like.

In particular, in the present invention, the tubular member is provided such that the longitudinal entirety of the tubular member is not fixed to the wire main body. The tubular member is provided such that the distal end portion of the tubular member is fixed or is not fixed to the distal end member. Accordingly, at least proximal end portion of the tubular member can rotate relative to the wire main body.

As a result, the guide wire of the present invention can be industrially applied.

### Reference Signs List

- 1:: guide wire
- 11:: wire main body
- 2:: first wire
- 21:: foremost end portion
- 22, 24:: tapered portion
- 23, 25:: constant outer-diameter portion
- 3:: second wire
- 4:: distal end member
- 41:: coil
- 42, 43:: fixing member
- 5:: tubular member
- 52:: distal-end side thickness reduction portion
- 53:: proximal-end side thickness reduction portion
- 54:: slit
- 55:: coil
- 56:: wire rod
- 57:: portion
- 6:: joined portion
- 7:: hydrophilic lubricating layer
- 8, 9:: resin coating layer
- W:: width
- φd:: diameter

## Claims

1. A guide wire comprising:
a wire main body that has flexibility;
a distal end member that is fixed to a distal end portion of the wire main body; and
a tubular member that is provided closer to a proximal end side than the distal end member, and on an outer circumference of the wire main body,
wherein the tubular member is provided such that a longitudinal entirety of the tubular member is not fixed to the wire main body.

2. The guide wire according to claim 1,
wherein the tubular member is provided such that a distal end portion of the tubular member is not fixed to the distal end member.

3. The guide wire according to claim 1 or 2,
wherein a proximal-end side thickness reduction portion with a wall thickness decreasing toward the proximal end side is provided in a proximal end portion of the tubular member.

4. The guide wire according to claim 3,
wherein an outer-diameter increasing portion with an outer diameter increasing toward the proximal end side is provided in a middle of the wire main body in a longitudinal direction of the wire main body, and
wherein in a side view of the wire main body, at least a portion of the proximal-end side thickness reduction portion overlaps the outer-diameter increasing portion.

5. The guide wire according to any one of claims 1 to 4,
wherein a distal-end side thickness reduction portion with a wall thickness decreasing toward a distal end side is provided in the distal end portion of the tubular member.

6. The guide wire according to any one of claims 1 to 5,
wherein the tubular member is provided with a cut-away portion that decreases the rigidity of the tubular member.

7. The guide wire according to claim 6,
wherein the cut-away portion is a slit that is helically provided around a center axis of the tubular member.

8. The guide wire according to any one of claims 1 to 5,
wherein the tubular member is configured as a coil that is made by helically winding a wire rod.

9. The guide wire according to claim 8,
wherein the number of turns of the wire rod per unit length of the coil in at least one of a distal end portion and a proximal end portion of the coil is less than the number of turns of the wire rod per unit length in an intermediate portion between the distal end portion and the proximal end portion of the coil.

10. The guide wire according to any one of claims 1 to 9,
wherein the distal end member is formed in a cylindrical shape, and
wherein the inner diameter of the tubular member is smaller than the outer diameter of the distal end member.

11. The guide wire according to any one of claims 1 to 10,
wherein the longitudinal length of the tubular member is greater than that of the distal end member.

12. The guide wire according to any one of claims 1 to 11,
wherein a slide resistance reduction member is provided between the tubular member and the wire main body so as to reduce slide resistance between the tubular member and the wire main body.

13. The guide wire according to claim 12,
wherein the slide resistance reduction member is made of a hydrophilic material, fluorine-based resin, or silicon-based resin.

14. The guide wire according to any one of claims 1 to 13,
wherein the distal end member includes the coil that is made by helically winding the wire rod.
